# EUROPEAN PATENT SPECIFICATION

(11) **EP 4 138 746 B1**
(45) Date of publication and mention of the grant of the patent: **24.12.2025**
(21) Application number: 21719729.2
(22) Date of filing: 12.04.2021
(51) Int. Cl.: A61F 9/007

(54) **MULTI-CHANNEL PIEZOELECTRIC RESONANT SYSTEM**
MEHRKANALIGES PIEZOELEKTRISCHES RESONANZSYSTEM
SYSTÈME RÉSONANT PIÉZOÉLECTRIQUE À CANAUX MULTIPLES

(30) Priority: 23.04.2020 US 202016856835
(43) Date of publication of application: 01.03.2023
(73) Proprietor: Johnson & Johnson Surgical Vision, Inc., Irvine, CA 92618 (US)
(72) Inventor: STEEN, Mark Evan, Santa Ana, California 92705 (US); GLINER, Vadim, 2066717 Yokneam (IL); GOVARI, Assaf, 2066717 Yokneam (IL)
(74) Representative: Carpmaels & Ransford LLP
(86) International application number: PCT/IB2021/053014
(87) International publication number: WO 2021/214589

(56) References cited:
- EP-A1- 1 849 444
- WO-A1-00/00298
- WO-A1-2013/173495
- US-A- 5 431 664

## Description

### FIELD OF THE INVENTION

The present invention relates generally to piezoelectric-vibration-based systems, and particularly to phacoemulsification systems.

### BACKGROUND OF THE INVENTION

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution to maintain the anterior chamber of the eye. After removing the cataract with phacoemulsification, the softer outer lens cortex is removed with suction. An intraocular lens (IOL) is then introduced into the empty lens capsule restoring the patient's vision.

Various techniques to vibrate a phacoemulsification needle of a probe were proposed in the patent literature. For example, U.S. Patent Application Publication 2010/0069825 describes a method and system for use in an ocular surgical procedure. The design includes a handpiece having an ultrasonically vibrating tip operational within a plurality of operating modes including a first operating mode and a sensing device, such as a vacuum pressure sensor. A controller is connected to the handpiece and sensing device and is configured to receive data from the sensing device and adjust at least one operational parameter (time/duty cycle of operation, power during operation) associated with the first operating mode and adjust at least one parameter associated with another operating mode based on the data received from the sensing device. Operational modes may include multiple longitudinal or non-longitudinal modes (torsional, transversal, etc.) or combinations of longitudinal and/or non-longitudinal modes.

As another example, U.S. Patent 8,303,613 describes a Langevin transducer horn that uses split electroding or selective electroding of transducer elements and phase relationships of the voltages applied thereto to determine the relative longitudinal and flexural/transverse motion induced in the tip of the horn. In an embodiment, an ultrasonic surgical instrument is provided, that includes a piezoelectric transducer element attached to the horn such that excitation of the piezoelectric element using one of the above electroding causes vibration of a working member of the horn.

U.S. Patent Application Publication 2008/0294087 describes phacoemulsification systems and methods, and more particularly systems and methods for providing transverse phacoemulsification. In accordance with one embodiment, a phacoemulsification system is provided having a handpiece with a needle, wherein the phacoemulsification system is configured to vibrate the needle in both an effective transverse direction and an effective longitudinal direction when power having a single effective operating frequency is applied to the handpiece.

U.S. Patent 8,623,040 describes a phacoemulsification cutting tip with a straight shaft and an angled portion off of the straight shaft that may include a hook on the angled portion to move an axis of rotation of the cutting tip closer to alignment with an extended centerline of the shaft. The cutting tip may be configured to torsionally rotate back and forth on an axis perpendicular to a centerline of the shaft (e.g., rotation around a y-axis). In some embodiments, lateral vibrations (e.g., side to side along an x-axis or z-axis perpendicular to the y-axis) that result from torsional rotation around the y-axis in a cutting tip without the hook may be reduced through use of the hook to balance the otherwise eccentrically weighted hook. In some embodiments, the cutting tip may be ultrasonically torsionally vibrated along a small arc (e.g., +/-5 degrees). The torsional vibrations of the cutting tip may result in lateral motions in the shaft and the cutting tip.

WO00/00298 provides an apparatus for tuning and controlling an ultrasonic handpiece having a programmable broad spectrum source, a torsional single frequency source and a longitudinal single frequency source that generate a drive signal for an ultrasonic handpiece and a digital signal processor for analyzing a response signal generated by the handpiece in response to the drive signal and generating and adjusting signal for adjusting the torsional single frequency source and the longitudinal single frequency source.

EP1849444 provides a system for controlling an ultrasonic handpiece of an ocular surgical system, such as a phacoemulsification system. First and second signal sources generate first and second drive signals. The first signal is at a first frequency and is used to drive a cutting tip of the handpiece with a first type of motion. The second signal is at a second frequency and is used to drive the cutting tip with a second type of motion. The different motions can be generated with different first and second frequencies. The first and second signals can be summed or combined and provided to a class D amplifier, the output of which includes multiple frequency components or multiple signals of different frequencies to drive the cutting tip in different directions at the same time, for example, with simultaneous longitudinal and torsional motions.

WO2013/173495 provides a piezoelectric ejector assembly, in which a piezoelectric actuator is attached to an ejector mechanism, while a drive signal generator and a controller are coupled to the actuator. The drive signal generator is configured to generate a drive signal for driving the actuator to oscillate the ejector assembly. The controller is configured to control the drive signal generator to drive the actuator at a resonant frequency of the ejector assembly, and an auto-tuning circuit is provided to define the optimum drive signal frequency.

### SUMMARY OF THE INVENTION

An embodiment of the present invention that is described hereinafter provides a multi-channel drive system for a piezoelectric actuator having a multiple-frequency resonant mode, the system including multiple signal generators, multiple feedback circuitries, and a processor. The multiple signal generators are configured to generate multiple respective drive signals at multiple respective drive signal frequencies, and to drive the piezoelectric actuator with the multiple drive signals. The multiple feedback circuitries are configured to measure multiple respective feedback signals at the multiple drive signal frequencies. The processor is configured to adaptively maintain the piezoelectric actuator vibrating in the multiple-frequency resonant mode, by adjusting the drive signal frequencies in response to the respective measured feedback signals. The multi-channel drive system further comprising a switching circuitry, wherein the piezoelectric actuator has one or more multiple-split electrodes disposed thereon. Each multiple - split electrode is formed of multiple electrode segments. The processor is configured to connect at least two of the drive signals to respective different combinations of the electrode segments. The processor is further configured to connect at least part of the multiple signal generators, using the switching circuitry, with different combinations of the one or more multiple-split electrodes.

In some embodiments, the processor is configured to adjust each of the drive signal frequencies independently of any other of the drive signal frequencies.

In some embodiments, the feedback signals include respective voltages of the drive signals across the piezoelectric actuator, and respective electrical currents flowing through the piezoelectric actuator in response to the drive signals, wherein the multiple feedback circuitries are configured to estimate respective phase differences between the respective voltages and the respective electrical currents.

In an embodiment, the processor is configured to adaptively adjust the drive signal frequencies so as to reduce the phase differences.

In another embodiment, the processor is configured to run a multiple-frequency proportional-integral-derivative (PID) control architecture to adjust the drive signal frequencies.

In some embodiments, the piezoelectric actuator is included in a phacoemulsification probe to drive a needle of the probe.

There is additionally described a multi-channel driving method not being part of this invention but serving explanatory reasons only. Said driving method for a piezoelectric actuator having a multiple-frequency resonant mode, the method including generating multiple respective drive signals at multiple respective drive signal frequencies. The piezoelectric actuator is driven with the multiple drive signals. Multiple respective feedback signals are measured at the multiple drive signal frequencies. The piezoelectric actuator is adaptively maintained vibrating in the multiple-frequency resonant mode, by adjusting the drive signal frequencies in response to the respective measured feedback signals.

There is further provided, in accordance with another embodiment of the present invention, a phacoemulsification apparatus, including a phacoemulsification probe, a piezoelectric actuator, and a multi-channel drive system. The phacoemulsification probe includes a needle configured for insertion into a lens capsule of a human eye. The piezoelectric actuator is configured to vibrate the needle and having a multiple-frequency resonant mode. The multi-channel drive system includes (a) multiple signal generators configured to generate multiple respective drive signals at multiple respective drive signal frequencies, and to drive the piezoelectric actuator with the multiple drive signals, (b) multiple feedback circuitries configured to measure multiple respective feedback signals at the multiple drive signal frequencies, and (c) a processor configured to adaptively maintain the piezoelectric actuator vibrating in the multiple-frequency resonant mode, by adjusting the drive signal frequencies in response to the respective measured feedback signals.

The invention is defined by the appended claims.

The present invention will be more fully understood from the following detailed description of the embodiments thereof, taken together with the drawings in which:

### BRIEF DESCRIPTION OF THE DRAWINGS

Fig. 1 is a pictorial view, along with a block diagram, of a phacoemulsification apparatus constructed and operating in accordance with an embodiment of the present invention;
Fig. 2 is a block diagram schematically describing the multi-channel piezoelectric drive system of the phacoemulsification apparatus of Fig. 1, in accordance with an embodiment of the present invention;
Fig. 3 is a flow chart schematically describing a method for operating the phacoemulsification apparatus of Fig. 1; and
Fig. 4 is a pictorial, schematic drawing of a multi-stack piezoelectric disposed with split electrodes that, using the multi-channel piezoelectric drive system of Fig. 2, can be driven using various possible coupling schemes, in accordance with embodiments of the present invention.

### DETAILED DESCRIPTION OF EMBODIMENTS

### OVERVIEW

A phacoemulsification system typically drives a piezoelectric actuator included in a phacoemulsification probe/handpiece to vibrate a needle of the phacoemulsification probe during a cataract procedure. The piezoelectric actuator of the phacoemulsification probe may be designed to vibrate, in resonance, in multiple modes simultaneously, where each mode has a given "natural" resonant frequency. For example, a multi-resonance mode might yield a complex vibration profile that combines longitudinal, transverse, and torsion vibrations, each with its own resonant frequency. Such a mode may have a complex customizable vibration profile that may allow a physician to better perform phacoemulsification.

However, interactions among the different vibration modes of the multi-mode vibration may change their natural frequencies, and the frequencies may further change, for example, as the crystal heats up when it is loaded by ocular media. Thus, it is difficult for the piezoelectric drive system to maintain all of the modes in resonance. The resonant frequency of each of the multiple modes may further depend upon other factors, such as the voltage and current amplitude applied to the piezoelectric actuator, and various other acoustic impedances encountered by the piezoelectric actuator. As a result, complex motion modes, and their potential benefits, may not be practically achievable or maintained for a sufficiently long time.

Moreover, if the resonant frequencies of the piezoelectric actuator change, either due to one of the factors described above, or for any other reason, and further, if the piezoelectric actuator is still powered with signals having the same frequencies (i.e., with part or all of the frequencies being off-resonance), the piezoelectric actuator will heat further. The additional heat may lead to further changes in the resonant frequencies, which in turn may lead to further heat, and so on. Such effects are further complicated because the various resonant frequencies of the piezoelectric actuator typically vary in a non-linear fashion and interact with each other.

Inadequate control of the vibration frequencies can therefore also lead to a hazard as the phacoemulsification needle becomes too hot for the eye. For example, the phacoemulsification needle could reach a temperature of 42 °C, above which the proteins in the eye may coagulate, which is very dangerous for the eye. While irrigation may be used to reduce the temperature of the phacoemulsification needle, irrigation presents its own problems. For example, irrigation without carefully matched aspiration can increase internal eye pressure to dangerous levels, whereas too much aspiration can lead to eye collapse. Moreover, irrigation may not be sufficient to adequately cool the phacoemulsification needle.

Embodiments of the present invention that are described hereinafter provide improved methods and systems for driving piezoelectric actuator in phacoemulsification applications. The disclosed techniques facilitate multi-resonant phacoemulsification vibration modes to improve probe efficacy and, at the same time, solve thermal hazard problems. Some embodiments provide a phacoemulsification apparatus comprising a multi-channel resonant drive system that drives the piezoelectric actuator (e.g., a piezoelectric crystal of the actuator) in a multimode vibration mode by adaptively adjusting each of the frequencies of the drive signals independently of the other frequencies. In this way, the drive system collectively (i.e., using all frequencies simultaneously) drives the piezoelectric actuator while tracking the changing resonant frequencies, thereby maximizing stroke amplitude, enabling a complex motion (e.g., vibration) profile of the needle (e.g., moving in an elliptical track) while minimizing temperature rise of the phacoemulsification probe.

The disclosed embodiments provide individual processor-controlled drive modules to drive each resonant-frequency mode of vibration while controlling the driving oscillator circuitry comprised in the drive-module to oscillate in resonance with the crystal mode it drives regardless of the aforementioned changes in the mode resonant frequency or in the resonant frequencies of other modes. Each of the separate drive modules may be realized in hardware or software, for example, in a proportional-integral-derivative (PID) control architecture. The different frequencies of the drive signals are adjusted independently of the others and enable vibration of the piezoelectric actuator continuously at the selected multimode resonant mode.

While driving the vibration, the drive modules modify the driving signal frequencies to follow the actuator's varying resonant frequencies by minimizing each frequency with a measured feedback signal, such as a measured phase difference between different voltages across the piezoelectric actuator and respective currents flowing through the piezoelectric actuator in response to the different drive signals. More formally, each module measures a phase difference, Δϕ, between the driving voltage V and the resulting current I outputted by the driving oscillator and minimize Δϕ, to maintain the oscillator driving the crystal mode in a resonance frequency. Each drive module thus maintains a nominal resonant frequency f*₁*, f*₂*, ..., f*_{N}*, and the different drive modules each vary a respective nominal frequency by minimizing respective phase difference, Δϕⱼ, j=1,2..., N, thereby keeping the complex-mode of the crystal in resonance.

In an embodiment, the phacoemulsification probe includes a horn, a needle coupled with the horn and configured for insertion into a lens capsule of an eye, and a piezoelectric actuator configured to vibrate the horn and the needle with a multiple-frequency resonant mode. The probe is driven by a multi-channel piezoelectric drive system, having multiple respective resonant drive signal frequencies, the system comprising: (a) multiple respective signal generators configured to generate the multiple respective drive signals to drive a vibration of a piezoelectric actuator at the multiple drive signal frequencies of a respectively multiple-frequency resonant mode of the piezoelectric actuator, (b) multiple respective phase detection circuitries configured to measure respective multiple phase differences between respective voltages of the drive signals across the piezoelectric actuator, and respective electrical currents flowing through the piezoelectric actuator in response to the drive signals delivered at the multiple drive signal frequencies, and (c) a processor configured to independently adjust each drive signal frequency so as to minimize the respective multiple measured phase differences, to maintain the piezoelectric actuator vibrating at the multiple-frequency resonant mode.

In some embodiments, the multimode piezoelectric crystal comprises a stack of crystals, whereas in other embodiments, a single crystal is used. The one or more crystals are terminated by a uniform or multiple-split electrode. In an embodiment, each multiple-split electrode is formed of multiple electrode segments, and the processor is configured to connect at least two of the drive signals to respective different combinations of the electrode segments. For example, four separate electrode segments may be comprised in a single electrode to allow the aforementioned multi-channel resonant drive system to vibrate any type of piezoelectric crystal in multiple modes, as described below. In addition, combinations of these modes may be used in synchrony to generate, for example, a final needle motion as the aforementioned elliptical track needle vibration.

By providing a phacoemulsification apparatus that drives multiple electrodes resonantly, and by using multiple drive modules to maintain the multi-resonant mode of motion, improved phacoemulsification may be possible.

### SYSTEM DESCRIPTION

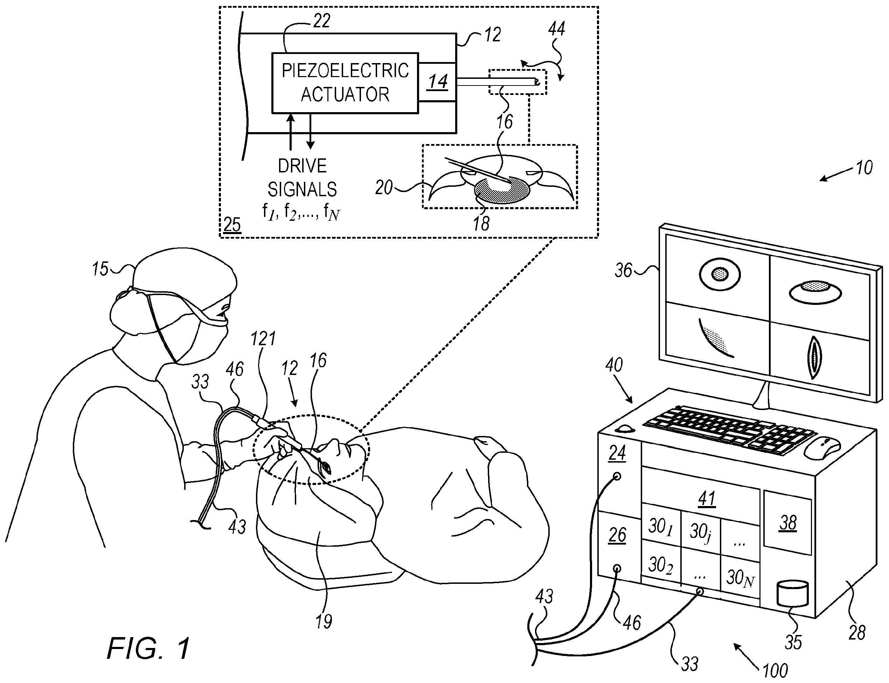

Fig. 1 is a pictorial view, including a block diagram, of a phacoemulsification apparatus 10 constructed to operate in accordance with an embodiment of the present invention. As seen in the pictorial view of phacoemulsification apparatus 10, and the block diagram in inset 25, it includes a phacoemulsification probe/handpiece 12 comprising a needle 16 configured for insertion into a lens capsule 18 of an eye 20 of a patient 19 by a physician 15. Needle 16 is mounted on a horn 14 of probe 12, and is shown in inset 25 as a straight needle. However, any suitable needle may be used with the phacoemulsification probe 12, for example, a curved or bent tip needle commercially available from Johnson & Johnson Surgical Vision, Santa Ana, CA, USA.

A piezoelectric actuator 22 is configured to vibrate horn 14 and needle 16 in one or more resonant vibration modes of the combined horn and needle element. The vibration of needle 16 is used to break a cataract into small pieces during the phacoemulsification procedure.

In the shown embodiment, during the phacoemulsification procedure, a pumping sub-system 24 comprised in a console 28 pumps irrigation fluid from an irrigation reservoir to needle 16 to irrigate the eye. The fluid is pumped via a tubing line 43 running from the console 28 to the probe 12. Waste matter (e.g., emulsified parts of the cataract) and eye fluid are aspirated via needle 16 to the collection receptacle by a pumping sub-system 26 also comprised in console 28 and using another tubing line 46 running from probe 12 to console 28.

Console 28 further comprises a multi-channel piezoelectric drive system 100 comprising drive-modules 30₁, 30₂, ... 30_{N}, each coupled, using electrical wiring running in cable 33, with a stack of piezoelectric crystals of actuator 22. Drive-modules 30₁, 30₂, ... 30_{N}, are controlled by a processor 38 and convey phase-controlled driving signals via cable 33 to adjust frequencies of a multi resonance mode of piezoelectric actuator 22. In response, actuator 22 vibrates needle 16, which performs a complex vibrational trajectory 44 comprising, for example, a combination of longitudinal, transverse, and/or torsional vibrations in synchronization one with the other.

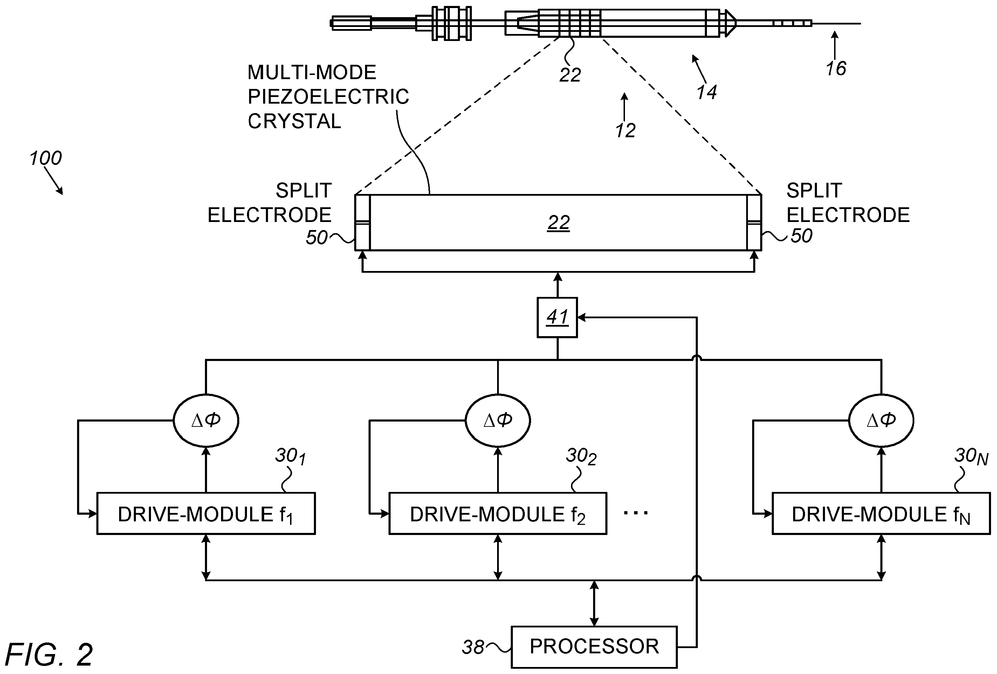

Processor 38 (shown in Fig. 2) adjusts the different frequencies *f*₁, *f*₂*,* ... *f*_{N} of the drive signals to minimize measured phase differences using any suitable method, for example, an optimization algorithm which is not limited to a gradient descent algorithm. An apparatus that can adjust a frequency of a drive signal so as to minimize the measured phase difference, whereby maintaining a piezoelectric actuator vibrating at a resonant frequency, is described in U.S. Patent Application 16/704054, filed December 5, 2019, titled "Phacoemulsification Apparatus," which is assigned to the assignee of the present patent application.

In an embodiment, piezoelectric actuator 22 is disposed with one or more multiple-split electrodes, and processor 38 is configured to connect different combinations of the one or more multiple-split electrodes, using a switching circuitry 41, to at least part of drive-modules 30₁, 30₂, ... 30_{N}, so as to vibrate needle 16 in synchrony with one of several possible prespecified trajectories, such as trajectory 44.

Some or all of the functions of processor 38 may be combined in a single physical component or, alternatively, implemented using multiple physical components. These physical components may comprise hard-wired or programmable devices, or a combination of the two. In some embodiments, at least some of the functions of processor 38 may be carried out by suitable software stored in a memory 35 (as shown in Fig. 1). This software may be downloaded to a device in electronic form, over a network, for example. Alternatively, or additionally, the software may be stored in tangible, non-transitory computer-readable storage media, such as optical, magnetic, or electronic memory.

Processor 38 may receive user-based commands via a user interface 40, which may include setting a vibration mode and/or frequency of the piezoelectric actuator 22, adjusting the vibration mode and/or frequency of the piezoelectric actuator 22, setting or adjusting a stroke amplitude of the needle 16, and setting or adjusting an irrigation and/or aspiration rate of the pumping sub-system 26. Additionally, or alternatively, processor 38 may receive user-based commands from controls located in handle 121, to, for example, select trajectory 44, or another trajectory, for needle 16.

Processor 38 is further configured to control the aforementioned pumping sub-systems 24 and 26. As seen in Fig. 1, processor 38 may present results of the procedure on a display 36. In an embodiment, user interface 40 and display 36 may be one and the same such as a touch screen graphical user interface.

The apparatus shown in Fig. 1 may include further elements, which are omitted for clarity of presentation. For example, physician 15 typically performs the procedure using a stereo-microscope or magnifying glasses, neither of which are shown. Physician 15 may use other surgical tools in addition to probe 12, which are also not shown to maintain clarity and simplicity of presentation.

### MULTI-CHANNEL PIEZOELECTRIC RESONANT SYSTEM FOR PHACOEMULSIFICATION PROBE

Fig. 2 is a block diagram schematically describing the multi-channel piezoelectric drive system 100 of phacoemulsification apparatus 10 of Fig. 1, in accordance with an embodiment of the present invention.

As seen, drive system 100 comprises drive-modules 30₁, 30₂, ... 30_{N}, each coupled to one or more split electrodes 50 of piezoelectric actuator 22 (which may comprise a multi-stack crystal) of phacoemulsification probe 12, using electrical links running in cable 33.

Drive-modules 30₁, 30₂, ... 30_{N}, convey driving signals having resonant frequencies *f*₁, *f*₂, ... *f*_{N} of a multi resonance mode of piezoelectric actuator 22 that drive-modules 30₁, 30₂, ... 30_{N}, controlled by processor 38, may adjust by minimizing detected respective phase differences, Δϕⱼ, j=1,2..., N, to keep the complex-mode of the crystal in resonance, e.g., following commands from the processor.

Processor 38 is further configured to connect at least part of drive-modules 30₁, 30₂, ... 30_{N}, using a switching circuitry 41, with different combinations of the one or more multiple-split electrodes 50 of piezoelectric actuator 22, so as to vibrate needle 16 in synchrony in one of several prespecified trajectories.

The example illustration shown in Fig. 2 is chosen purely for the sake of conceptual clarity. Fig. 2 shows only parts relevant to embodiments of the present invention. Other system elements, such as for eye irrigation, and for removal of debris from the eye, are omitted.

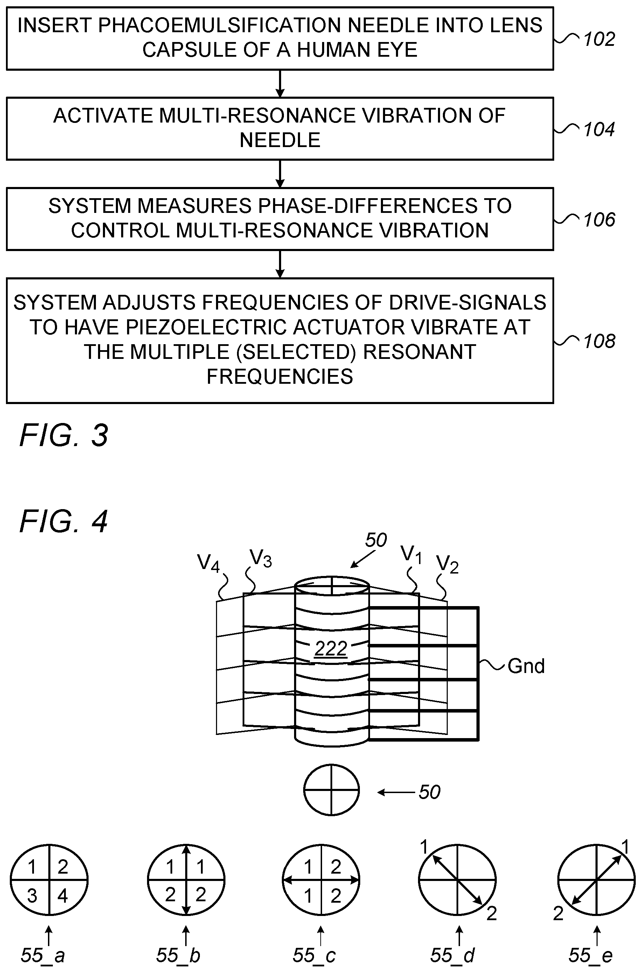

Fig. 3 is a flow chart schematically describing a method for operating phacoemulsification apparatus 10 of Fig. 1. The algorithm, according to the presented embodiment, carries out a process that begins with physician 15 inserting phacoemulsification needle 16 of probe 12 into a lens capsule 18 of an eye 20, at a probe insertion step 102.

Next, physician 15 activates, for example using a control over handle 121 or a foot pedal (not shown), probe 12 to vibrate needle 16 in complex trajectory 44, at a needle vibrating step 104. In response, processor 38 commands a multi-channel piezoelectric drive system 100 to generate signals to drive piezoelectric actuator 22 in the selected multi-resonance vibration mode.

At a needle vibration controlling step 106, drive-modules 30₁, 30₂, ... 30_{N} measure the aforementioned phase differences between voltages and currents across and through piezoelectric actuator 22 (e.g., between split electrodes 50).

Finally, at a needle motion control step 108, system 100 uses the phase information control step 106 to adjust frequencies of the drive signals such that piezoelectric actuator 22 vibrates at the multiple (selected) resonant frequencies, so as to continue vibrating needle 16 in complex trajectory 44.

The example flow chart shown in Fig. 3 is chosen purely for the sake of conceptual clarity. For example, additional steps such as cutting, irrigating, and inspecting the eye are omitted for simplicity and clarity of presentation.

Fig. 4 is a pictorial, schematic drawing of a multi-stack piezoelectric 222 disposed with split electrodes 50 that, using multi-channel piezoelectric drive system 100 of Fig. 2, can be driven using various possible coupling schemes 55_a-55_e, in accordance with embodiments of the present invention.

As Fig. 4 shows, each of electrodes 50 is split into four electrode segments that receive respective voltages V₁-V₄ relative to an electrical ground, Gnd.

The 4-split electrodes enable various driving configurations, as follows:
Configuration 55_a, in which all four electrode segments are independently driven by four different voltages, e.g., voltages generated by four respective drive-modules 30₁-30₄ at different (e.g., similar but not necessarily equal) resonant frequencies. By selecting synchronization and amplitude of voltages V₁-V₄, a complex vibration trajectory, such as a circular trajectory, is possible with configuration 55_a.

Configuration 55_b has two electrode segments that are independently driven by two different voltages to vibrate a crystal in one lateral axis, whereas configuration 55_c has two electrode segments independently driven by two different voltages to vibrate the crystal in an orthogonal lateral direction relative that of to 55_b.

Configurations 55_d and 55_e have two electrode segments independently driven by two different voltages to vibrate a crystal in two mutually orthogonal lateral axes that are rotated 45° relative to those of configurations 55_b and 55_c.

The spilt electrodes of Fig. 4 are brought by way of example, and other configurations are possible, such as having six electrode segments, each with a 60° angular section of an electrode 50.

Although the embodiments described herein mainly address phacoemulsification, the methods and systems described herein can also be used in other applications that may require a multi-channel piezoelectric resonant system to drive a moving member.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. The scope of the present invention is defined by the appended claims.

## Claims

1. A multi-channel drive system (100) for a piezoelectric actuator (22) having a multiple-frequency resonant mode, the system comprising:
multiple signal generators (30) configured to generate multiple respective drive signals at multiple respective drive signal frequencies, and to drive the piezoelectric actuator (22) with the multiple respective drive signals;
multiple feedback circuitries configured to measure multiple respective feedback signals at the multiple respective drive signal frequencies;
a processor (38) configured to adaptively maintain the piezoelectric actuator vibrating in the multiple-frequency resonant mode, by adjusting the respective drive signal frequencies in response to the respective measured feedback signals; and
a switching circuitry (41); wherein
the piezoelectric actuator has one or more multiple-split electrodes (50) disposed thereon, each multiple-split electrode formed of multiple electrode segments, and wherein the processor is configured to connect at least two of the drive signals to respective different combinations of the electrode segments,
wherein the processor (38) is further configured to connect at least part of the multiple signal generators, using the switching circuitry (41), with different combinations (55) of the one or more multiple-split electrodes (50).

2. The multi-channel drive system (100) according to claim 1, wherein the processor (38) is configured to adjust each of the multiple respective drive signal frequencies independently of any other of the multiple respective drive signal frequencies.

3. The multi-channel drive system (100) according to claim 1, wherein the feedback signals comprise respective voltages of the drive signals across the piezoelectric actuator (22), and respective electrical currents flowing through the piezoelectric actuator in response to the multiple respective drive signals, wherein the multiple feedback circuitries are configured to estimate respective phase differences between the respective voltages and the respective electrical currents.

4. The multi-channel drive system (100) according to claim 3, wherein the processor (38) is configured to adaptively adjust the multiple respective drive signal frequencies so as to reduce the phase differences.

5. The multi-channel drive system (100) according to claim 1, wherein the processor (38) is configured to run a multiple-frequency proportional-integral-derivative (PID) control architecture to adjust the multiple respective drive signal frequencies.

6. The multi-channel drive system (100) according to claim 1, wherein the piezoelectric actuator (22) is comprised in a phacoemulsification probe (12) to drive a needle (16) of the probe.

7. A phacoemulsification apparatus (10), comprising:
a phacoemulsification probe (12) comprising a needle (16) configured for insertion into a lens capsule of an eye;
a piezoelectric actuator (22) configured to vibrate the needle and having a multiple-frequency resonant mode; and
the multi-channel drive system (100) of any of claims 1 to 6.

8. The phacoemulsification apparatus (10) according to claim 7, wherein the respective measured feedback signals comprise respective voltages of the drive signals across the piezoelectric actuator, and respective electrical currents flowing through the piezoelectric actuator in response to the drive signals, and wherein the multiple feedback circuitries are configured to estimate respective phase differences between the respective voltages and the respective electrical currents.

## Patentansprüche

1. Mehrkanal-Antriebssystem (100) für einen piezoelektrischen Aktuator (22) mit einem Mehrfrequenz-Resonanzmodus, wobei das System umfasst:
mehrere Signalgeneratoren (30), die konfiguriert sind, um mehrere jeweilige Antriebssignale bei mehreren jeweiligen Antriebssignalfrequenzen erzeugen und den piezoelektrischen Aktuator (22) mit den mehreren jeweiligen Antriebssignalen anzutreiben;
mehrere Rückkopplungsschaltkreise, die konfiguriert sind, um mehrere jeweilige Rückkopplungssignale bei den jeweiligen Antriebssignalfrequenzen zu messen;
einen Prozessor (38), der konfiguriert ist, um den piezoelektrischen Aktuator adaptiv in dem Mehrfrequenz-Resonanzmodus schwingen zu lassen, indem er die jeweiligen Antriebssignalfrequenzen als Reaktion auf die jeweiligen gemessenen Rückkopplungssignale anzupassen; und
einen Schaltkreis (41); wobei
der piezoelektrische Aktuator eine oder mehrere mehrfach geteilte Elektroden (50) aufweist, die darauf angeordnet sind, wobei jede mehrfach geteilte Elektrode aus mehreren Elektrodensegmenten besteht, und wobei der Prozessor konfiguriert ist, um mindestens zwei der Antriebssignale mit jeweils unterschiedlichen Kombinationen der Elektrodensegmente zu verbinden,
wobei der Prozessor (38) ferner konfiguriert ist, um mittels des Schaltkreises (41) mindestens einen Teil der mehreren Signalgeneratoren mit verschiedenen Kombinationen (55) der einen oder mehreren mehrfach geteilten Elektroden (50) zu verbinden.

2. Mehrkanal-Antriebssystem (100) nach Anspruch 1, wobei der Prozessor (38) konfiguriert ist, um jede der mehreren jeweiligen Antriebssignalfrequenzen unabhängig von jeder anderen der mehreren jeweiligen Antriebssignalfrequenzen anzupassen.

3. Mehrkanal-Antriebssystem (100) nach Anspruch 1, wobei die Rückkopplungssignale die jeweiligen Spannungen der Antriebssignale an dem piezoelektrischen Aktuator (22), und die jeweiligen elektrischen Ströme umfassen, die als Reaktion auf die mehreren Antriebssignale durch den piezoelektrischen Aktuator fließen, wobei die mehreren Rückkopplungsschaltkreise konfiguriert sind, um die jeweiligen Phasendifferenzen zwischen den jeweiligen Spannungen und den jeweiligen elektrischen Strömen zu schätzen.

4. Mehrkanal-Antriebssystem (100) nach Anspruch 3, wobei der Prozessor (38) konfiguriert ist, um die mehreren Antriebssignalfrequenzen adaptiv anzupassen, um die Phasendifferenzen zu verringern.

5. Mehrkanal-Antriebssystem (100) nach Anspruch 1, wobei der Prozessor (38) konfiguriert ist, um eine Mehrfrequenz-Proportional-Integral-Differential (PID)-Regelarchitektur auszuführen, um die mehreren Antriebssignalfrequenzen anzupassen.

6. Mehrkanal-Antriebssystem (100) nach Anspruch 1, wobei der piezoelektrische Aktuator (22) in einer Phakoemulsifikationssonde (12) zum Antrieb einer Nadel (16) der Sonde umfasst ist.

7. Phakoemulsifikationseinrichtung (10), umfassend:
eine Phakoemulsifikationssonde (12), umfassend eine Nadel (16), die zum Einführen in die Linsenkapsel eines Auges konfiguriert ist;
einen piezoelektrischen Aktuator (22), der konfiguriert ist, um die Nadel in Schwingung zu versetzen, und der über einen Mehrfrequenz-Resonanzmodus verfügt; und
das Mehrkanal-Antriebssystem (100) nach einem der Ansprüche 1 bis 6.

8. Phakoemulsifikationseinrichtung (10) nach Anspruch 7, wobei die jeweiligen gemessenen Rückkopplungssignale die jeweiligen Spannungen der Antriebssignale an dem piezoelektrischen Aktuator und die jeweiligen elektrischen Ströme umfassen, die als Reaktion auf die Antriebssignale durch den piezoelektrischen Aktuator fließen, und wobei die mehreren Rückkopplungsschaltkreise konfiguriert sind, um die jeweiligen Phasendifferenzen zwischen den jeweiligen Spannungen und den jeweiligen elektrischen Strömen zu schätzen.

## Revendications

1. Système d'entraînement multicanal (100) pour un actionneur piézoélectrique (22) ayant un mode de résonance à multiples fréquences, le système comprenant :
de multiples générateurs de signaux (30) configurés pour générer de multiples signaux d'entraînement respectifs à de multiples fréquences de signal d'entraînement respectives, et pour entraîner l'actionneur piézoélectrique (22) avec les multiples signaux d'entraînement respectifs ;
de multiples circuits de rétroaction configurés pour mesurer de multiples signaux de rétroaction respectifs aux multiples fréquences de signal d'entraînement respectives ;
un processeur (38) configuré pour maintenir de manière adaptative l'actionneur piézoélectrique vibrant dans le mode de résonance à multiples fréquences, en ajustant les fréquences de signal d'entraînement respectives en réponse aux signaux de rétroaction mesurés respectifs ; et
un circuit de commutation (41) ; dans lequel
l'actionneur piézoélectrique a une ou plusieurs électrodes à divisions multiples (50), disposées sur celui-ci, chaque électrode à divisions multiples étant formée de multiples segments d'électrode, et dans lequel le processeur est configuré pour connecter au moins deux des signaux d'entraînement à différentes combinaisons respectives des segments d'électrode,
dans lequel le processeur (38) est en outre configuré pour connecter au moins une partie des multiples générateurs de signaux, à l'aide du circuit de commutation (41), à différentes combinaisons (55) de la ou des électrodes à divisions multiples (50).

2. Système d'entraînement multicanal (100) selon la revendication 1, dans lequel le processeur (38) est configuré pour ajuster chacune des multiples fréquences de signal d'entraînement respectives indépendamment de l'une quelconque des autres multiples fréquences de signal d'entraînement respectives.

3. Système d'entraînement multicanal (100) selon la revendication 1, dans lequel les signaux de rétroaction comprennent des tensions respectives des signaux d'entraînement aux bornes de l'actionneur piézoélectrique (22), et des courants électriques respectifs circulant à travers l'actionneur piézoélectrique en réponse aux multiples signaux d'entraînement respectifs, dans lequel les multiples circuits de rétroaction multiples sont configurés pour estimer des déphasages respectifs entre les tensions respectives et les courants électriques respectifs.

4. Système d'entraînement multicanal (100) selon la revendication 3, dans lequel le processeur (38) est configuré pour ajuster de manière adaptative les multiples fréquences de signal d'entraînement respectives de manière à réduire les déphasages.

5. Système d'entraînement multicanal (100) selon la revendication 1, dans lequel le processeur (38) est configuré pour exécuter une architecture de commande proportionnelle-intégrale-dérivée (PID) à fréquences multiples afin d'ajuster les multiples fréquences de signal d'entraînement respectives.

6. Système d'entraînement multicanal (100) selon la revendication 1, dans lequel l'actionneur piézoélectrique (22) est compris dans une sonde de phacoémulsification (12) pour entraîner une aiguille (16) de la sonde.

7. Appareil de phacoémulsification (10), comprenant :
une sonde de phacoémulsification (12) comprenant une aiguille (16) conçue pour une insertion dans la capsule du cristallin d'un oeil ;
un actionneur piézoélectrique (22) configuré pour faire vibrer l'aiguille et ayant un mode de résonance à fréquences multiples ; et
le système d'entraînement multicanal (100) selon l'une quelconque des revendications 1 à 6.

8. Appareil de phacoémulsification (10) selon la revendication 7, dans lequel les signaux de rétroaction mesurés respectifs comprennent les tensions respectives des signaux d'entraînement aux bornes de l'actionneur piézoélectrique, et des courants électriques respectifs circulant à travers l'actionneur piézoélectrique en réponse aux signaux d'entraînement, et dans lequel les multiples circuits de rétroaction sont configurés pour estimer des déphasages respectives entre les tensions respectives et les courants électriques respectifs.
